# EUROPEAN PATENT APPLICATION

(11) **EP 4 732 749 A1**
(43) Date of publication of application: **29.04.2026**
(21) Application number: 24879957.9
(22) Date of filing: 06.09.2024
(51) Int. Cl.: A61B 1/005, A61B 1/00

(54) **ENDOSCOPE**

(30) Priority: 16.10.2023 KR 20230137466
(71) Applicant: LG Electronics Inc., Yeongdeungpo-gu Seoul 07336 (KR)
(72) Inventor: HWANG, Changkyu, Seoul 06772 (KR); KIM, Wonsu, Seoul 06772 (KR); KIM, Soonbeom, Seoul 06772 (KR)
(74) Representative: Vossius & Partner Patentanwälte Rechtsanwälte mbB
(86) International application number: PCT/KR2024/013543
(87) International publication number: WO 2025/084618

(57) **Abstract**

The present invention relates to an endoscope including an insertion part provided with a lighting/imaging part and an insertion pipe, and a manipulation part provided with a manipulation knob and configured to bend the insertion pipe in accordance with a manipulation of the manipulation knob, in which the insertion pipe includes a bending part configured to be bent in accordance with a manipulation of the manipulation part, and in which the bending part includes a first bending member and a second bending member that are alternately disposed in a coil spring and configured to rotate relative to each other, and the first and second bending members are formed in female-male shapes and guide coupling positions, such that the plurality of bending members may be simply assembled without a separate component, and the shape thereof may be maintained even in the assembled state.

## Description

### [BACKGROUND]

### [Technical Field]

The present invention relates to an endoscope, and more particularly, to an endoscope in which an insertion part and a manipulation part may be detachably provided, and the disposable insertion part may be used and replaced.

### [Background Art]

A medical endoscope relates to a device configured to be inserted directly into internal tissue of a human body, which is a target to be subjected to examinations or procedures, and used to identify states of diseased parts or perform procedures.

A general endoscope has an articulated assembly disposed at an end of a flexible insertion tube, and the articulated assembly enables an insertion direction to be adjusted during a process of inserting the endoscope into a human body. A leading end body, which is equipped with an image sensor and a light source, is provided at an end of the articulated assembly. A handle body may be provided at the other end of the insertion tube. The handle body is connected to a non-illustrated image processing device through a separate cable and a separate connector.

Dials and buttons, which are used to adjust a direction of the articulated assembly and perform the procedure, are disposed on the handle body.

The dials may be provided as a pair of dials and used to adjust a traveling direction of the leading end body upward, downward, leftward, and rightward with respect to a longitudinal direction of the insertion tube. A sprocket and a chain are provided in the handle body and convert a rotational motion of the dial into a linear motion. The chain is connected to an end of the articulated assembly through a separate wire. As a result, the rotational motion of the dial is converted into the linear motion of the wire by means of the sprocket and the chain, such that the articulated assembly is bent by the linear motion of the wire. Therefore, an operator of the endoscope may adjust the traveling direction of the leading end body during the process of inserting the insertion tube into the human body.

In this case, because the endoscope is inserted into the human body, thorough hygiene management is required, and the endoscope needs to be necessarily washed/disinfected before and after treatment or surgery. In some instances, it may be impossible or undesirable to reuse parts, which are actually inserted into the human body, i.e., the insertion tube and the articulated assembly. In this case, because the entire endoscope in the related art is integrated, there occurs a problem in that the entire endoscope needs to be replaced. In addition, even in case that the part of the endoscope may be reused, the washing and disinfection operations may be conveniently performed in case that only some parts of the endoscope may be separated without disassembling the entire endoscope.

Meanwhile, Japanese Patent No. JP 6401429 B2 discloses an endoscope including a bending part configured to be bent by a plurality of bending pieces.

However, because the bending piece of the endoscope is formed in an annular shape having three thicknesses, it is difficult to manufacture the bending piece.

In addition, there is no structure capable of guiding accurate positions of four wires even though it is necessary for the four wires to pass through the plurality of bending pieces and be assembled, which makes it difficult to perform an assembling process and maintain a shape in the assembled state.

### [DETAILED DESCRIPTION OF THE INVENTION]

### [Technical Problem]

The present invention is proposed to solve these problems of the endoscope in the related art and aims to provide an endoscope in which a plurality of bending members may be simply assembled without a separate component.

The present invention also aims to provide an endoscope capable of being prevented from being damaged by gaps occurring between a plurality of bending members during a bending operation.

### [Technical Solution]

In order to achieve the above-mentioned objects, an endoscope according to the present invention includes: an insertion part provided with a lighting/imaging part and an insertion pipe; and a manipulation part provided with a manipulation knob and configured to bend the insertion pipe in accordance with a manipulation of the manipulation knob, in which the insertion pipe includes a bending part configured to be bent in accordance with a manipulation of the manipulation part, and in which the bending part includes a first bending member and a second bending member that are alternately disposed in a coil spring and configured to rotate relative to each other.

Specifically, the bending part may include a first bending member formed with a concave portion; and a second bending member formed with a convex portion and fastened to the first member.

Therefore, the concave portion and the convex portion may guide a coupling position.

In this case, the first bending member may include an annular first bending member body formed with the concave portion, and the concave portion may be provided as a pair of concave portions respectively formed at two opposite longitudinal ends of the first bending member body.

Therefore, the first bending member may guide all the coupling positions of the second bending members disposed forward and rearward in the longitudinal direction.

In addition, the pair of concave portions may be formed at positions that face each other in a circumferential direction at the two opposite longitudinal ends of the first bending member body.

That is, the concave portions may be disposed with a predetermined phase difference in a circumferential direction of the first bending member body.

Meanwhile, the second bending member may include a second bending member body having a longitudinal end from which the convex portion protrudes, and the convex portion may be relatively rotatably accommodated in the concave portion.

Therefore, the first bending member and the second bending member may rotate about the pair of concave portions as rotation centers.

Meanwhile, the insertion part may further include a bending wire configured to bend the insertion pipe while rectilinearly reciprocating, and the second bending member may further include a wire guide groove formed in an inner surface of the convex portion and configured to restrict a bending angle of the bending wire.

Therefore, it is possible to restrict a bending angle of the bending part and prevent damage caused by the occurrence of excessive bending.

Meanwhile, a wire through-hole through which the bending wire passes may be disposed radially inside the concave portion or the convex portion.

Meanwhile, the insertion pipe may further include adapters coupled to the first bending member or the second bending member and disposed at a longitudinal end of the bending part.

In this case, the adapter may include: an adapter body formed in a cylindrical shape; and a wire accommodation groove formed in a longitudinal direction of the adapter body and configured to accommodate at least a part of the bending wire.

Meanwhile, the insertion pipe may further include a coil spring configured to accommodate therein the bending part.

Therefore, it is possible to apply a restoring force to the bending of the bending part. In case that excessive bending occurs, the restoring force may be increased to prevent damage.

Meanwhile, two opposite longitudinal ends of the coil spring may be coupled to adapters coupled to two opposite longitudinal ends of the bending part.

### [Advantageous Effects]

According to the endoscope according to the present invention described above, the pair of bending members are formed in female-male shapes and guide the coupling positions, such that the plurality of bending members may be simply assembled without a separate component, and the shape thereof may be maintained even in the assembled state.

In addition, the coil spring may be provided on the outer peripheral surface of the bending part. Therefore, even though a gap occurs between the plurality of bending members during the bending operation, the coil spring may restore the bending members by applying an elastic force to the portion where an excessive gap occurs, thereby preventing the occurrence of damage.

### [Description of Drawings]

FIG. 1 is a perspective view for explaining an endoscope according to an embodiment of the present invention.
FIG. 2 is a perspective view for explaining a detachable relationship between a manipulation part and an insertion part of the endoscope according to the embodiment of the present invention.
FIG. 3 is a top plan view of FIG. 2.
FIG. 4 is a view for explaining a method of coupling a first coupling part and a second coupling part of the endoscope according to the embodiment of the present invention.
FIG. 5 is an enlarged view of FIG. 4 when viewed at another angle.
FIG. 6 is a view for explaining a state in which the first coupling part and the second coupling part of the endoscope according to the embodiment of the present invention are coupled.
FIGS. 7 to 13 are views for explaining a structure for guiding an initial position of a wire frame of the endoscope according to the embodiment of the present invention.
FIG. 14 is a perspective view of FIG. 2 when viewed at another angle.
FIG. 15 is a perspective view for explaining a lighting/imaging part of the endoscope according to the embodiment of the present invention.
FIG. 16 is a perspective view for explaining an internal structure of the lighting/imaging part of the endoscope according to the embodiment of the present invention.
FIG. 17 is a view for explaining a heat radiation plate of the endoscope according to the embodiment of the present invention.
FIGS. 18 and 19 are views for explaining an insertion pipe of the endoscope according to the embodiment of the present invention.
FIG. 20 is a view for explaining a first bending member of the endoscope according to the embodiment of the present invention.
FIG. 21 is a view for explaining a second bending member of the endoscope according to the embodiment of the present invention.

### [Description of Embodiments]

Hereinafter, exemplary embodiments of the present invention will be described in detail with reference to the accompanying drawings.

The present invention may be variously modified and may have various embodiments, and particular embodiments illustrated in the drawings will be specifically described below. The description of the embodiments is not intended to limit the present invention to the particular embodiments, but it should be interpreted that the present invention is to cover all modifications, equivalents and alternatives falling within the spirit and technical scope of the present invention.

In the description of the present invention, the terms such as "first" and "second" may be used to describe various constituent elements, but the constituent elements may not be limited by the terms. These terms are used only to distinguish one constituent element from another constituent element. For example, a first component may be named a second component, and similarly, the second component may also be named the first component, without departing from the scope of the present invention.

The term "and/or" may include any and all combinations of a plurality of the related and listed items.

When one constituent element is described as being "coupled" or "connected" to another constituent element, it should be understood that one constituent element can be coupled or connected directly to another constituent element, and an intervening constituent element can also be present between the constituent elements. When one constituent element is described as being "coupled directly to" or "connected directly to" another constituent element, it should be understood that no intervening constituent element is present between the constituent elements.

The terminology used herein is used for the purpose of describing particular embodiments only and is not intended to limit the present invention. Singular expressions may include plural expressions unless clearly described as different meanings in the context.

The terms "comprises," "comprising," "includes," "including," "containing," "has," "having" or other variations thereof are inclusive and therefore specify the presence of stated features, integers, steps, operations, elements, components, and/or combinations thereof, but do not preclude the presence or addition of one or more other features, integers, steps, operations, elements, components, and/or combinations thereof.

Unless otherwise defined, all terms used herein, including technical or scientific terms, may have the same meaning as commonly understood by those skilled in the art to which the present invention pertains. The terms such as those defined in a commonly used dictionary may be interpreted as having meanings consistent with meanings in the context of related technologies and may not be interpreted as ideal or excessively formal meanings unless explicitly defined in the present application.

Further, the following embodiments are provided to more completely explain the present invention to those skilled in the art, and shapes and sizes of elements illustrated in the drawings may be exaggerated for a more apparent description.

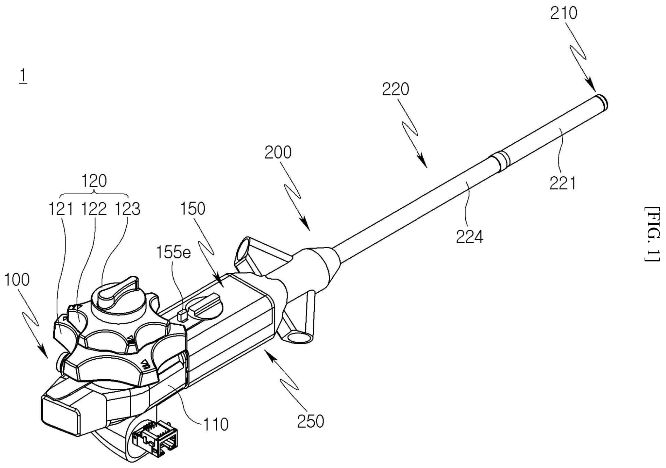

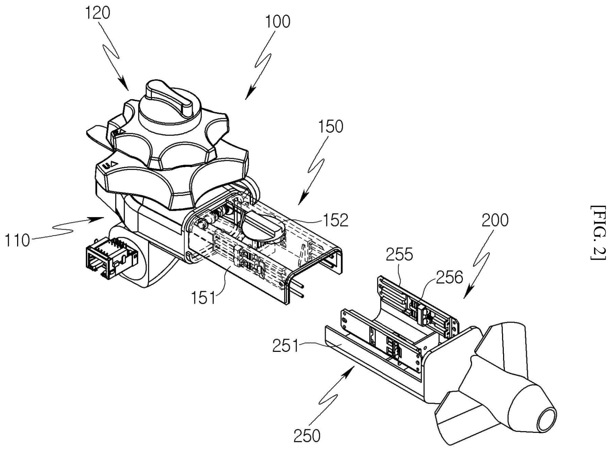

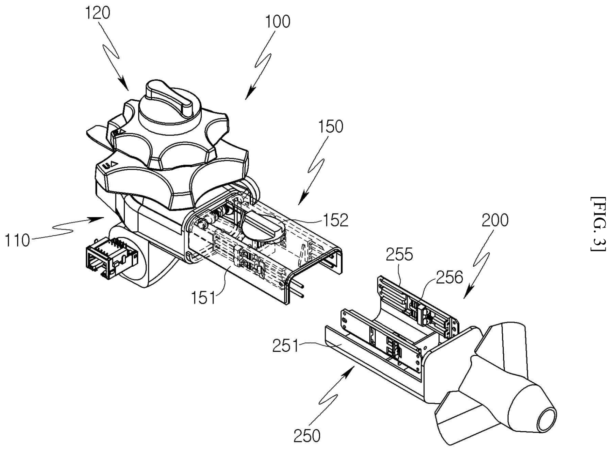

FIG. 1 is a perspective view for explaining an endoscope according to an embodiment of the present invention, FIG. 2 is a perspective view for explaining a detachable relationship between a manipulation part and an insertion part of the endoscope according to the embodiment of the present disclosure, and FIG. 3 is a top plan view of FIG. 2.

With reference to FIGS. 1 to 3, an endoscope 1 according to an embodiment of the present invention may be a disposable endoscope. Specifically, the endoscope 1 according to the embodiment of the present invention includes a manipulation part 100 and an insertion part 200. In this case, the manipulation part 100 and the insertion part 200 may be detachably coupled to each other by coupling parts 150 and 250. In this case, the coupling parts 150 and 250 include a first coupling part 150 provided in the manipulation part 100, and a second coupling part 250 provided in the insertion part 200.

The coupling parts 150 and 250 in the present invention are configured to convert a rotational motion of a dial 152 into a rectilinear motion by means of a pinion gear 153 and rack gears 154. Therefore, the coupling parts 150 and 250 are configured such that coupling plates 155 and 255 respectively provided in the manipulation part 100 and the insertion part 200 are coupled to each other. In this case, the coupling plates 155 and 255 include first coupling plates 155 provided in the manipulation part 100, and second coupling plates 255 provided in the insertion part 200.

Meanwhile, in the present invention, wires 130 and 230 may be connected by wire frames 156 and 256. That is, the wires 130 includes manipulation wires 130 provided in the manipulation part 100, and bending wires 230 provided in the insertion part 200, and the wires are coupled to first wire frames 156, which are provided in the manipulation part, and second wire frames 256 provided in the insertion part 200. In this case, the first wire frame 156 and the second wire frame 256 may be detachably coupled to each other. In the state in which the first wire frame 156 and the second wire frame 256 are coupled, the first wire frame 156 and the second wire frame 256 may rectilinearly reciprocate in the coupling plates 155 and 255.

This configuration will be described below in detail.

The manipulation part 100 is provided to be manipulated by an operator. The manipulation part 100 may be provided to be gripped and manipulated by the operator. The operator may manipulate the manipulation part 100 to bend the insertion part 200.

The insertion part 200 is provided to be inserted into a human body by the manipulation of the manipulation part 100 and to capture images of the inside of the human body. The insertion part 200 may be inserted into the human body and provided to be bendable by the manipulation of the manipulation part 100, such that the insertion part 200 may move along an internal structure of the human body, illuminate the inside of the human body with light, and capture images of the inside of the human body.

Although not illustrated, in addition to the above-mentioned components, the endoscope 1 according to the embodiment of the present invention may further include a connector adapter and a universal cord.

Specifically, the endoscope 1 may include a connector adapter configured to be connected to an external device, and the connector adapter may be connected to the manipulation part 100 by the universal cord. The external device connected to the connector adapter may be an image processing device. Therefore, the image captured by the insertion part 200 may be displayed on an external image processing device. Therefore, the operator may manipulate the manipulation part 100 while observing the image processing device that displays the image captured by the insertion part 200.

The manipulation part 100 includes a manipulation part housing 110, a manipulation knob 120, the manipulation wire 130, and the first coupling part 150.

The manipulation part housing 110 may provide a structure that may be gripped by a practitioner, and the manipulation part housing 110 may accommodate therein the manipulation wire 130 or the like configured to operate the insertion part 200.

Specifically, the manipulation knob 120 may be disposed on an upper portion of the manipulation part housing 110, and the first coupling part 150 may be disposed at one longitudinal side of the manipulation knob 120. Sprockets, chains, and the manipulation wire 130, which operate in conjunction with a rotation of the manipulation knob 120, may be accommodated in the manipulation part housing 110, and other components, such as a channel, may further be accommodated in the manipulation part housing 110.

The manipulation knob 120 may control the insertion part 200 while being rotated by the manipulation by the user. The manipulation knob 120 may include two or more knobs. For example, the manipulation knob 120 may be provided in a shape in which a first knob 121 and a second knob 122 are stacked in an upward/downward direction. The first knob 121 and the second knob 122 may be respectively connected to a first sprocket and a second sprocket that will be described below, and the first knob 121 and the second knob 122 may be configured to rotate independently of each other. The first knob 121 and the second knob 122 may each have a shape in which a plurality of teeth protrude so that the practitioner may easily grip and rotate the first knob 121 and the second knob 122.

Meanwhile, the first knob 121 may rotate independently while the second knob 122 is kept stopped. Likewise, the second knob 122 may rotate independently while the first knob 121 is kept stopped. The first knob 121 controls upward and downward movements of a lighting/imaging part 210. That is, when the first knob 121 rotates clockwise or counterclockwise, an end of the lighting/imaging part 210 is bent upward or downward. The second knob 122 controls leftward and rightward movements of the lighting/imaging part 210. That is, when the second knob 122 rotates clockwise or counterclockwise, the end of the lighting/imaging part 210 is bent leftward or rightward.

As described above, the rotation direction of the lighting/imaging part 210 may be arbitrarily adjusted by manipulating the first knob 121 and the second knob 122. According to the embodiment, it may be necessary to fix a state in which the lighting/imaging part 210 is bent at a particular angle. To this end, a fixing knob 123 is provided above the second knob 122. When the fixing knob 123 is rotated, the first sprocket and the second sprocket are fixed so as not to rotate, such that the end of the lighting/imaging part 210 may also be kept fixed.

Although not illustrated, two sprockets are mounted below the first knob 121 and the second knob 122 and configured to be rotatable in conjunction with the rotations of the first and second knobs 121 and 122. Further, the chains, which engage with the respective sprockets and move, are provided. The chains may perform the upward and downward movements and the leftward and rightward movements of the lighting/imaging part 210, and the chains may move independently of each other.

Two opposite ends of the chain are coupled to the manipulation wire 130. The movement of the manipulation wire 130 is guided by a guide structure provided in the manipulation part housing 110.

The manipulation wire 130 is coupled to the first coupling part 150. Specifically, the manipulation wire 130 may be coupled by the first wire frame 156 of the first coupling part 150. The first wire frame 156 is slidably mounted within a limited range of the first coupling plate 155.

Meanwhile, the first wire frames 156 may be coupled to the second wire frames 256 of the insertion part 200, such that the manipulation wires 130 may be connected to the bending wires 230.

Therefore, the manipulation wire 130 may bend an insertion pipe 220 in accordance with the manipulation of the manipulation knob 120.

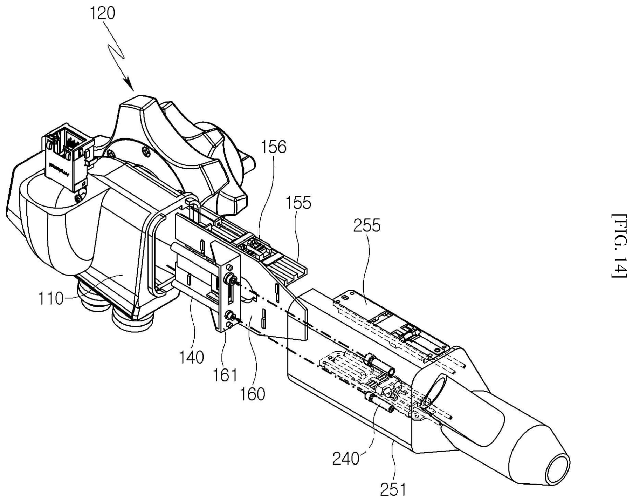

Meanwhile, FIG. 14 is a perspective view of FIG. 2 when viewed at another angle.

With reference to FIG. 14, the manipulation part 100 may be provided with channels 140 configured to provide air and water to a diseased part during a procedure. For example, the channels 140 may include a supply channel and a suction channel. In this case, the supply channel may be an air supply channel and/or a water supply channel. The air supply channel, the water supply channel, and the suction channel may each have an approximately cylindrical shape and have an empty interior. The air supply channel, the water supply channel, or the suction channel is inserted into the lighting/imaging part 210 when the lighting/imaging part 210 is coupled, such that air or water may be stably supplied to a site subjected to a procedure or examination without leaking.

Meanwhile, although not illustrated, the manipulation part 100 may further be provided with a connection terminal. The connection terminal may be provided to be electrically connected to the lighting/imaging part 210 so that data obtained by a camera or the like provided at the end of the lighting/imaging part 210 may be transmitted to the external device. In one example, the connection terminal may be an HDMI terminal configured to transmit voices and images.

The channel 140 and the connection terminal may be disposed below the wires 130 and 230. For example, the channel 140 and the connection terminal may be accommodated in a space, which is defined by the manipulation part housing 110, first coupling part bodies 151, and second coupling part bodies 251, and disposed to be farther from the manipulation knob 120 than the manipulation wire 130.

In this case, a region, in which the manipulation wire 130 and the bending wire 230 are moved, and a region, in which the channel 140 and the connection terminal are disposed, may be distinguished by a support plate 160. The support plate 160 may divide an internal space of the manipulation part 100 in the upward/downward direction and be provided with a channel support part 161 coupled to the channels 140 to support the channels 140, such that the support plate 160 may guide the connection of the channels 140 to exact positions when the manipulation part 100 and the insertion part 200 are coupled. That is, when the first coupling part 150 and the second coupling part 250 are coupled, the channels 140 of the manipulation part 100 may be connected to channels 240 of the insertion part 200.

The channel support part 161 may extend downward from a lower surface of the support plate 160 and be formed with circular holes so that the channels 140 are supported by the circular holes while penetrating the circular holes. In this case, the number of holes may correspond to the number of channels 140.

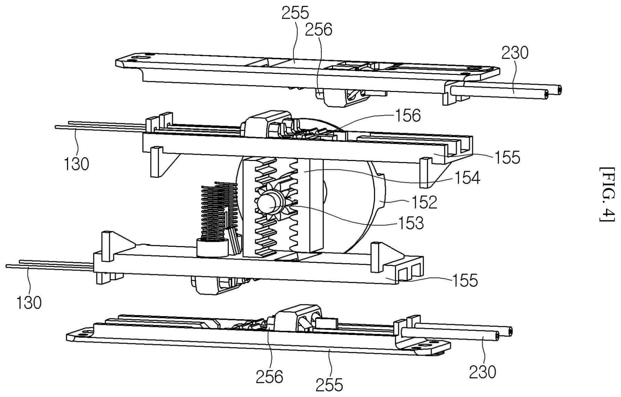

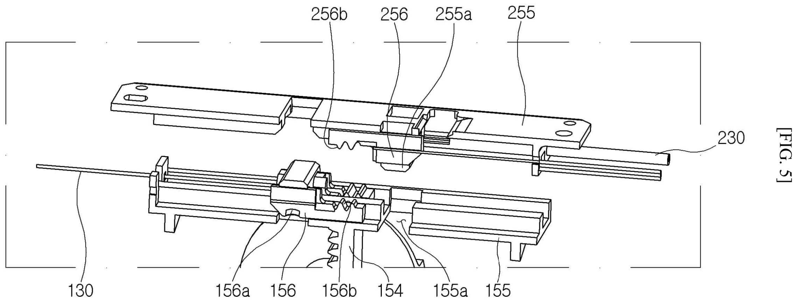

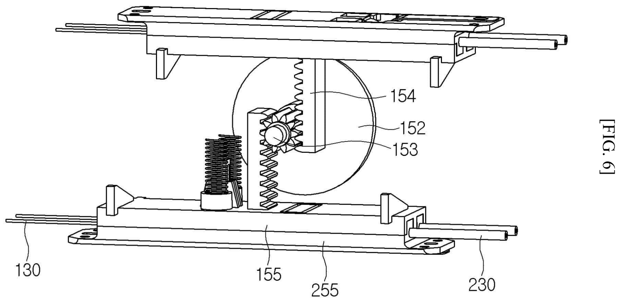

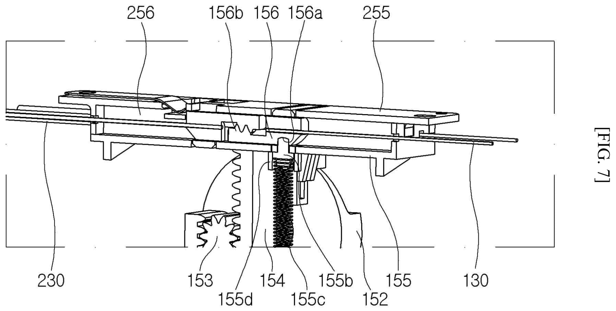

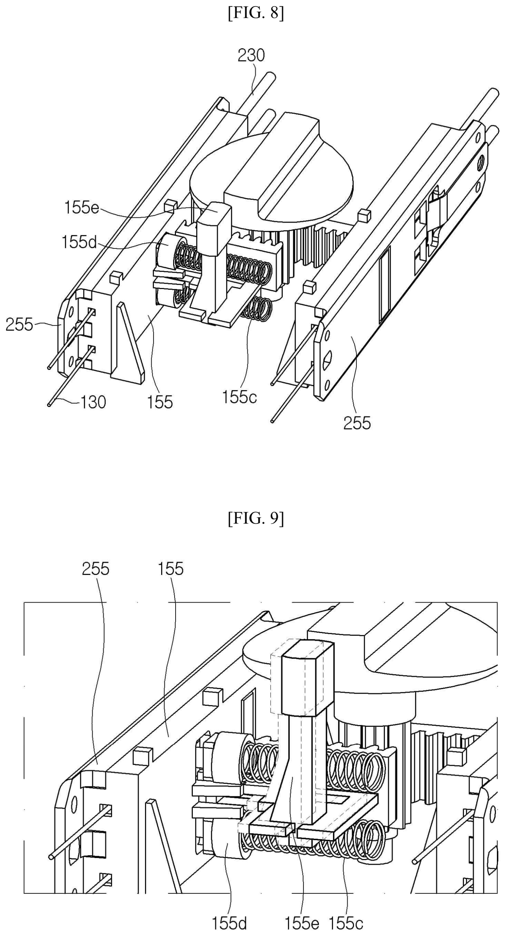

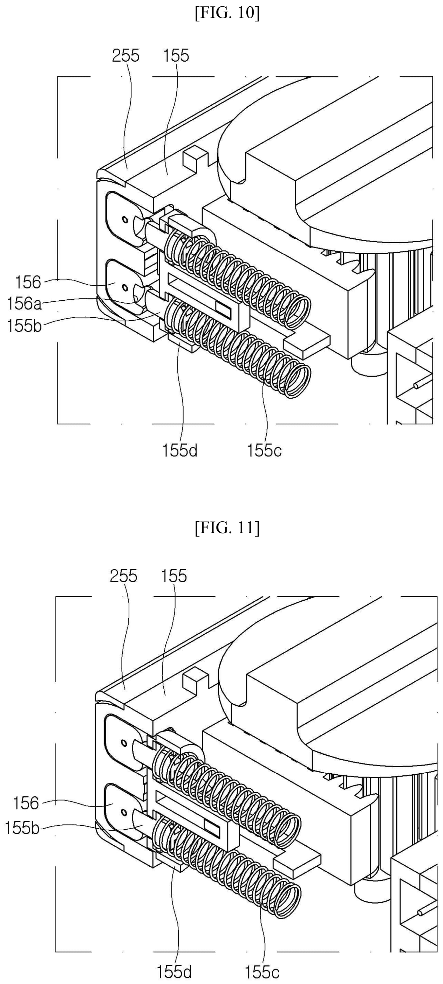

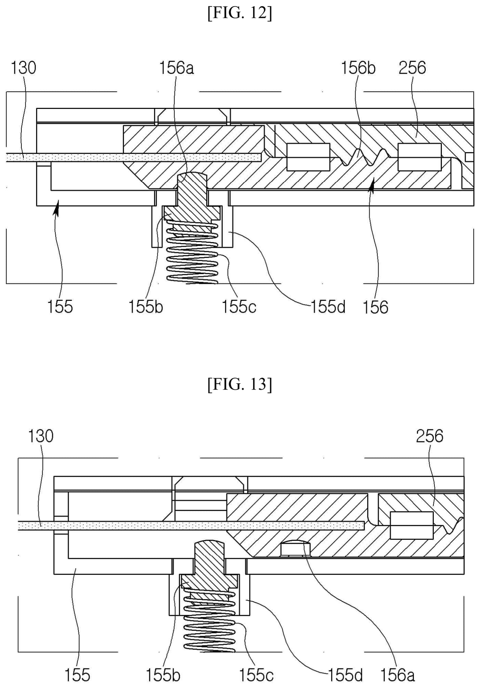

Meanwhile, FIG. 4 is a view for explaining a method of coupling the first coupling part and the second coupling part of the endoscope according to the embodiment of the present invention, FIG. 5 is an enlarged view of FIG. 4 when viewed at another angle, FIG. 6 is a view for explaining a state in which the first coupling part and the second coupling part of the endoscope according to the embodiment of the present invention are coupled, and FIGS. 7 to 13 are views for explaining a structure for guiding an initial position of the wire frame of the endoscope according to the embodiment of the present invention.

The first coupling part 150 will be described below with reference to FIGS. 4 to 13.

The first coupling part 150 may be disposed at one end of the manipulation part housing 110. The first coupling part 150 may be detachably coupled to the insertion part 200 by the manipulation by the practitioner (user).

Specifically, the first coupling part 150 includes the first coupling part bodies 151, the dial 152, the pinion gear 153, the rack gears 154, and the first coupling plates 155.

The first coupling part bodies 151 may be coupled by engaging with the second coupling part 250 of the insertion part 200. In one example, the first coupling part body 151 may be provided in the form of a plate that surrounds three sides. In another example, the first coupling part bodies 151 may be provided in the form of a pair of plates extending from the manipulation part housing 110 toward the insertion part 200. Therefore, the first coupling part bodies 151 may be coupled to the second coupling part bodies 251 of the second coupling part 250.

As a result, the first coupling part bodies 151 may be fitted with the second coupling part bodies 251.

The dial 152 may be configured such that the user may grip and rotate the dial 152. The dial 152 may be rotatably coupled to the manipulation part housing 110.

Specifically, the dial 152 may be exposed to the outside of the manipulation part housing 110. For example, the dial 152 may include a rotary plate formed in the shape of a circular plate, and a grip portion protruding in a rib shape from the rotary plate so that the user may hold and rotate the grip portion.

The pinion gear 153 may be disposed inside the first coupling part bodies 151 and rotated in conjunction with the rotation of the dial 152.

Meanwhile, the pinion gear 153 may be connected to the dial 152. The pinion gear 153 may pass through the first coupling part bodies 151 and be connected to the dial 152. For example, the pinion gear 153 and the dial 152 may be integrated.

Gear teeth may be formed on an outer peripheral surface of the pinion gear 153 and engage with the rack gears 154 to be described below. In this case, the pinion gear 153 may engage with the pair of rack gears 154. The pair of rack gears 154 may be positioned at positions that face each other based on the pinion gear 153.

The rack gear 154 may engage with the pinion gear 153 and rectilinearly move in accordance with the rotation of the pinion gear 153. In this case, the pair of rack gears 154, which face each other, may rectilinearly move in opposite directions in accordance with the rotation of the pinion gear 153.

The pair of rack gears 154 may be disposed between the pair of first coupling part bodies 151. In this case, the pair of rack gears 154 may be disposed in a direction intersecting a direction in which the first coupling part bodies 151 are formed, and the pair of rack gears 154 may rectilinearly reciprocate in the direction intersecting the direction in which the first coupling part bodies 151 are formed.

The pair of rack gears 154 may be respectively coupled to the first coupling plates 155. The first coupling plate 155 may be formed in a direction intersecting a longitudinal direction of the rack gear 154.

The first coupling plates 155 may be disposed between a pair of surfaces of the first coupling part bodies 151 that face each other. Further, the first coupling plates 155 may be disposed between the pair of second coupling part bodies 251. Meanwhile, the first coupling plates 155 may be disposed inside the second coupling part bodies 251 and disposed at positions that face the second coupling part bodies 251.

In addition, the first coupling plates 155 may be disposed at positions that face the second coupling plates 255. The first coupling plates 155 may move together with the rack gears 154 in accordance with the movements of the rack gears 154 and be coupled to the second coupling plates 255.

For example, the first coupling plate 155 may be formed in the shape of a quadrangular plate having a predetermined thickness. In this case, fixing portions 155a may be formed on the first coupling plates 155 to fix the state in which the first coupling plates 155 are coupled to the second coupling plates 255. In one example, the fixing portion 155a may be formed in the shape of a hole and coupled to a fixing portion 255a formed in the shape of a protrusion formed on the second coupling plate 255.

Meanwhile, the first wire frames 156 may be disposed in the first coupling plates 155. The first wire frame 156 may be coupled to the first coupling plate 155 and configured to rectilinearly reciprocate. For example, the first coupling plate 155 may be formed with a guide groove that guides the rectilinear movement of the first wire frame 156, and the first wire frame 156 may be disposed in the guide groove.

Meanwhile, the manipulation wire 130 is coupled to the first wire frame 156. For example, the manipulation wire 130 may be coupled to one longitudinal side of the first wire frame 156, and the first wire frame 156 may be configured to rectilinearly reciprocate in the longitudinal direction.

Meanwhile, the first wire frames 156 disposed in the first coupling plates 155 may be coupled to the second wire frames 256 disposed in the second coupling plates 255. For example, frame coupling parts 156b may each be formed at the other longitudinal side of each of the first wire frames 156. The frame coupling part 156b may have a shape in which a plurality of ribs and a plurality of grooves are alternately formed. The frame coupling parts 156b may be shaped to correspond to frame coupling parts 256b of the second wire frames 256. That is, the first wire frame 156 and the second wire frame 256 may be coupled in a state in which the first wire frame 156 and the second wire frame 256 engage with each other.

The first wire frame 156 may be disposed between the first coupling plate 155 and the second coupling plate 255 in the state in which the first wire frame 156 is coupled to the second wire frame 256. Therefore, in the state in which the first wire frame 156 and the second wire frame 256 are coupled to each other, the first wire frame 156 and the second wire frame 256 may rectilinearly reciprocate in the longitudinal direction of the endoscope 1.

Therefore, the manipulation wire 130 coupled to the first wire frame 156 and the bending wire 230 coupled to the second wire frame 256 may move in conjunction with each other, and the insertion pipe 220 may be bent by the manipulation of the manipulation knob 120.

Meanwhile, the first coupling plate 155 may further be provided with locking pins 155b, springs 155c, and locking holders 155d.

The locking pins 155b may be disposed on the first coupling plate 155, and the locking pins 155b may be accommodated in fixing grooves 156a formed in the first wire frames 156. The locking pin 155b may include a head portion accommodated in the locking holder 155d to be described below, and a pin portion passing through the locking holder 155d and accommodated in the fixing groove 156a. In this case, the head portion may be formed in a shape larger in diameter than the pin portion. Therefore, a movement range of the locking pin 155b may be restricted by the locking holder 155d.

Meanwhile, the locking pin 155b may be pressed toward the first wire frame 156 by the spring 155c. Specifically, the head portion of the locking pin 155b may be in contact with one end of the spring 155c. Therefore, the locking pin 155b may be elastically supported by the spring 155c.

The other end of the spring 155c may be fixed to the first coupling part body 151.

The locking holder 155d may be detachably coupled to the first coupling plate 155 and accommodate the locking pin 155b so that the locking pin 155b may reciprocate.

The locking holder 155d may be coupled to a locking lever 155e, and the locking holder 155d may be reciprocated by the operation of the locking lever 155e. In this case, the locking holder 155d may be detachably coupled to the first coupling plate 155 in accordance with the operation of the locking lever 155e.

The locking holder 155d may be formed to accommodate therein the locking pin 155b. For example, the locking holder 155d may be formed in a cylindrical shape. One longitudinal side of the locking holder 155d may be formed to correspond in size to the head portion of the locking pin 155b, and the other longitudinal side of the locking holder 155d may be formed to correspond in size to the pin portion of the locking pin 155b. That is, one longitudinal side of the locking holder 155d may be formed to be larger than the diameter of the head portion, and the other longitudinal side of the locking holder 155d may be formed to be smaller than the diameter of the head portion and larger than the diameter of the pin portion.

Therefore, the pin portion of the locking pin 155b may pass through the locking holder 155d and be accommodated in the fixing groove 156a of the first wire frame 156.

In one example, the locking holders 155d may be provided as a pair of locking holders 155d, and a lever coupling part coupled to the locking lever 155e may be provided between the pair of locking holders 155d. The lever coupling part 155e may be formed in the shape of a long hole, and one side of the locking lever 155e may be coupled to the lever coupling part 155e.

Meanwhile, one side of the locking lever 155e may be coupled to the locking holder 155d, and the other side of the locking lever 155e may be exposed to the outside of the first coupling part body 151. Therefore, the user may hold and manipulate the locking lever 155e.

Meanwhile, the first wire frame 156 may be formed with the fixing groove 156a. The fixing groove 156a may be formed at a side opposite to a side at which the frame coupling part 156b is formed. That is, the frame coupling part 156b may be formed at a position that faces the second wire frame 256, and a fixing groove 156a may be formed at a side opposite to the frame coupling part 156b.

The fixing groove 156a may accommodate the locking pin 155b in accordance with the movement of the first wire frame 156. That is, in case that the first wire frame 156 is disposed at a preset initial position, the fixing groove 156a may be disposed to face the locking pin 155b. In this case, when the user manipulates the locking lever 155e, the locking pin 155b may move along the locking holder 155d, pass through the first coupling plate 155, and be inserted into the fixing groove 156a.

Therefore, according to the present invention, after the insertion part 200 is replaced, the user may rotate the manipulation knob 120 in a state in which the user has operated the locking lever 155e. Therefore, the first wire frame 156 may rectilinearly move (slide) and find a position at which the locking pin 155b and the fixing groove 156a are coupled.

As a result, in the present invention, it is possible to find and maintain the initial position of the manipulation wire 130 after the insertion part 200 is replaced.

The insertion part 200 may be detachably coupled to the manipulation part 100, at least a part of the insertion part 200 may be inserted into the human body, and the insertion part 200 may capture images of the inside of the human body.

The insertion part 200 includes the lighting/imaging part 210, the insertion pipe 220, the bending wires 230, and the second coupling part 250. In this case, the lighting/imaging part 210 is disposed at one longitudinal side of the insertion part 200, and the second coupling part 250 is disposed at the other side of the insertion part 200. In addition, the lighting/imaging part 210 and the second coupling part 250 may be connected to the insertion pipe 220, and at least a part of the bending wire 230 may pass through the second coupling part 250 and the insertion pipe 220 from the second coupling part 250 to the insertion pipe 220.

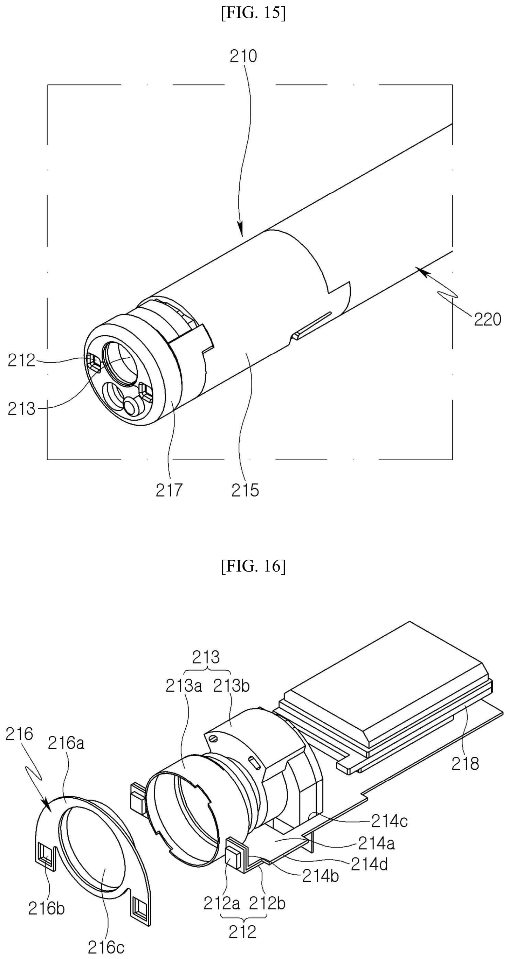

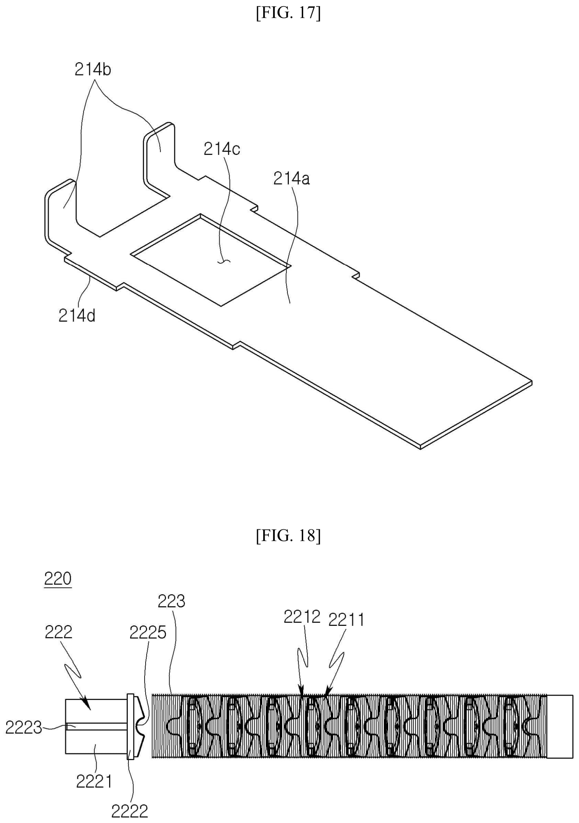

FIG. 15 is a perspective view for explaining the lighting/imaging part of the endoscope according to the embodiment of the present invention, FIG. 16 is a perspective view for explaining an internal structure of the lighting/imaging part of the endoscope according to the embodiment of the present disclosure, and FIG. 17 is a view for explaining a heat radiation plate of the endoscope according to the embodiment of the present invention.

The lighting/imaging part will be described below with reference to FIGS. 15 to 17.

The lighting/imaging part 210 may be inserted into the human body, illuminate the inside of the human body with light, and capture images of the inside of the human body.

The lighting/imaging part 210 includes a lighting/imaging part body 211, a lighting module 212, and an imaging module 213.

The lighting/imaging part body 211 is configured to define an external appearance of the lighting/imaging part 210 and be inserted into the human body. For example, the lighting module 212 and the imaging module 213 may be disposed at one longitudinal side of the lighting/imaging part body 211, and the other longitudinal side of the lighting/imaging part body 211 may be opened and connected to the insertion pipe 220.

The lighting module 212 may be coupled to an end cap 217. The lighting module 212 may emit light. The lighting module 212 may be provided with a lighting lens 212a capable of emitting light by receiving electric power. For example, the lighting lens 212a may be a light-emitting diode (LED).

Meanwhile, in the present embodiment, the lighting lens 212a may be provided as a plurality of lighting lenses 212a. Specifically, in the present embodiment, the lighting lenses 212a may be provided in an even number. For example, the lighting lenses 212a may be provided as a pair of lighting lenses disposed to be linearly symmetrical. Therefore, it is possible to prevent illuminance of an object created at a short distance from being asymmetrical and prevent gaps in lighting.

In this case, in the present embodiment, the pair of lighting lenses 212a may be disposed symmetrically based on the imaging module 213. That is, in the present embodiment, the pair of lighting lenses 212a may be disposed symmetrically, and the imaging module 213 may be disposed on a symmetric axis.

In this case, the pair of lighting lenses 212a may be different in correlated color temperature (CCT) from each other. Therefore, it is possible to create a targeted wavelength distribution of light.

In addition, the lighting module 212 may further include lighting support parts 212b configured to support the pair of lighting lenses 212a. The lighting support part 212b may be formed in the shape of a flat plate and have a pair of leading end portions extending symmetrically and bent upward. The pair of lighting lenses 212a may be coupled to the pair of bent extension portions.

For example, the lighting support part 212b may be a flexible printed circuit board (PCB). An image sensor may be mounted on the flexible PCB. Because the plurality of lighting lenses 212a are used for the lighting part, it is possible to easily adjust the illuminance and significantly increase the amount of light emission in comparison with a halogen lamp or the like in the related art. Therefore, it is possible to further improve the clarity of images obtained by the image sensor.

Meanwhile, the imaging module 213 may capture images of the inside of the human body. The imaging module 213 may capture images of the inside of the human body by receiving power and transmit the captured image data. For example, the imaging module 213 may include a camera 213a and an image processor 213b. In this case, the camera 213a may be disposed at one longitudinal end (leading end) of the lighting/imaging part 210, and the image processor 213b may be coupled to the camera 213a and process the image obtained by the camera 213a.

Therefore, in the state in which the endoscope 1 of the present invention is inserted into the human body, the lighting module 212 may emit light, and the imaging module 213 may capture images of the inside of the human body.

A heat radiation plate 214 may be disposed in the lighting/imaging part body 211. The heat radiation plate 214 may be coupled to the lighting module 212 and/or the imaging module 213.

The heat radiation plate 214 includes a heat radiation plate body 214a, lighting contact portions 214b, an imaging module accommodation hole 214c, and heat transfer portions 214d.

The heat radiation plate body 214a may be configured to transfer heat. The heat radiation plate body 214a may be made of a material capable of transferring heat. For example, the heat radiation plate body 214a may be made of a metallic material.

The heat radiation plate body 214a may be formed in the shape of a flat plate. One end of the heat radiation plate body 214a based on the longitudinal direction (major axis direction) may be connected to the lighting contact portions 214b, the heat transfer portions 214d are connected to two opposite sides of the heat radiation plate body 214a based on the width direction (minor axis direction), and the imaging module accommodation hole 214c may be formed in the heat radiation plate body 214a.

The lighting contact portion 214b may extend from one longitudinal end of the heat radiation plate body 214a and be in contact with the lighting module 212. The lighting contact portion 214b may be formed to correspond in shape to the lighting support part 212b. For example, the lighting contact portions 214b may extend from a leading end of the heat radiation plate body 214a based on the longitudinal direction and then bent and extending upward. Therefore, the lighting contact portions 214b may be in contact with the lighting support parts 212b. Therefore, the lighting contact portions 214b may transfer heat, which is generated from the lighting module 212, to the heat radiation plate 214.

The imaging module accommodation hole 214c may be formed in the heat radiation plate body, and the imaging module 213 may be penetratively accommodated in the imaging module accommodation hole 214c. For example, the imaging module accommodation hole 214c may be formed in the shape of a rectangular hole. A part of the image processor 213b may pass through the imaging module accommodation hole 214c, and a part of the image processor 213b may be in contact with the imaging module accommodation hole 214c. Therefore, the heat, which is generated from the imaging module 213, may be transferred to the heat radiation plate 214.

Meanwhile, a printed circuit board 218 may be in contact with the other longitudinal side of the heat radiation plate 214. The printed circuit board 218 may control the lighting module 212 and/or the imaging module 213. A circuit capable of controlling the lighting module 212 and/or the imaging module 213 may be mounted on the printed circuit board 218. Therefore, heat, which is generated from the circuit, may be transferred to the heat radiation plate 214.

The heat transfer portions 214d may extend from two opposite ends of the heat radiation plate body 214a based on the width direction (minor axis direction). In this case, a width of the heat radiation plate body 214a may be smaller than a diameter of a heat radiation chassis 215, and a sum of a width of the heat radiation plate body 214a and a width of the heat transfer portion 214d may be equal to or slightly larger than a diameter of the heat radiation chassis 215. Therefore, the heat transfer portion 214d may be in contact with the heat radiation chassis 215. Therefore, the heat of the heat transfer portions 214d may be transferred to the heat radiation chassis 215.

The heat radiation chassis 215 may be configured to surround an outer peripheral surface of the lighting/imaging part body 211, and the heat radiation chassis 215 may be in contact with the heat radiation plate 214 and disperse the heat from the lighting module 212 and/or the imaging module 213.

For example, the heat radiation chassis 215 may be a coil pipe made of a spring material and discharge the heat generated from the lighting module 212 and/or the imaging module 213.

Meanwhile, in another embodiment of the present invention, the heat radiation chassis 215 and the lighting/imaging part body 211 may be formed integrally.

Meanwhile, the lighting/imaging part 210 may further include a heat blocking plate 216 disposed at one longitudinal side of the lighting/imaging part 210 and configured to block the heat generated from the lighting module 212.

The heat blocking plate 216 may be coupled to the end cap 217 and disposed between the end cap 217 and the lighting module 212 in the longitudinal direction of the lighting/imaging part 210.

The heat blocking plate 216 may include a blocking plate body 216a formed in a semicircular shape, lighting cover portions 216b symmetrically disposed on the blocking plate body 216a and disposed to face the lighting module 212, and a camera cover portion 216c disposed on a symmetric axis of the pair of lighting cover portions 216b and disposed at positions that face the imaging module 213.

The heat blocking plate 216 may prevent the heat generated from the lighting module 212 from being transferred to the human body. In this case, the heat blocking plate 216 may be made of a material that may transmit light.

The end cap 217 is provided at the longitudinal end (leading end) of the lighting/imaging part 210. The end cap 217 has a cylindrical structure and is coupled to an end of the insertion part 200. In this case, the lighting module 212 and the imaging module 213 may be coupled to the end cap 217. In addition, the heat blocking plate 216 may be coupled to the end cap 217.

Light-transmitting windows may be provided at a plurality of points on the end cap 217. The light-transmitting window is configured as a board made of glass or transparent material and has a structure capable of transmitting the light emitted from the lighting module 212 to the outside.

Meanwhile, the end cap 217 may be formed with a plurality of channel accommodation pipes into which the channels 240 are inserted. The channel accommodation pipes each have a hollow cylindrical shape and have an inner diameter corresponding to an outer diameter of the channel 140 to be inserted. Further, an O-ring may be provided to prevent a leak of supplied water or air.

Meanwhile, the lighting/imaging part 210 may be provided with the connection terminal. The connection terminal may be provided to correspond to the connection terminal of the manipulation part 100. That is, the manipulation part 100 may be provided with a male terminal, and the insertion part 200 may be provided with a female terminal.

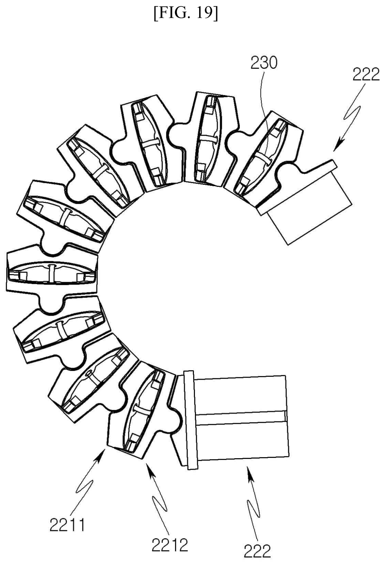

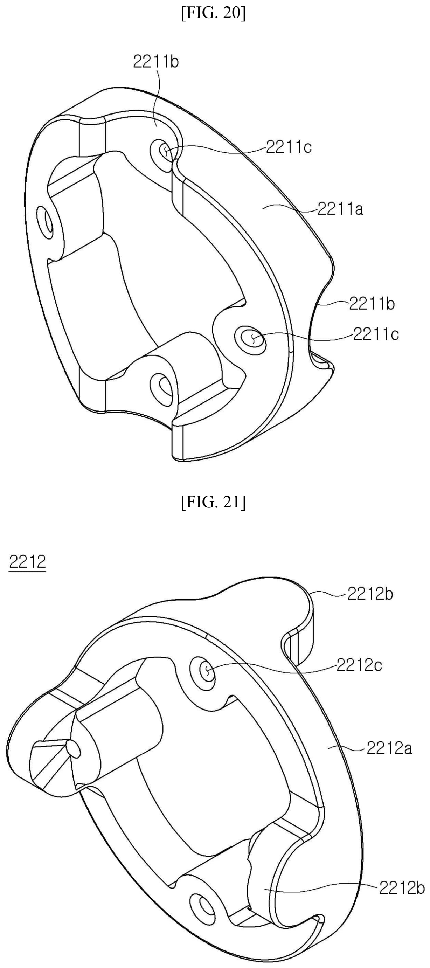

Meanwhile, FIGS. 18 and 19 are views for explaining the insertion pipe of the endoscope according to the embodiment of the present invention, FIG. 20 is a view for explaining a first bending member of the endoscope according to the embodiment of the present invention, and FIG. 21 is a view for explaining a second bending member of the endoscope according to the embodiment of the present invention.

The insertion pipe 220 will be described below with reference to FIGS. 18 to 21.

The insertion pipe 220 may connect the lighting/imaging part 210 and the second coupling part 250 and be bent in accordance with the manipulation of the manipulation part 100.

Specifically, the insertion pipe 220 may be bent by the movement of the bending wire 230.

Meanwhile, the plurality of channels 240 and the terminals may be accommodated in the insertion pipe 220.

The insertion pipe 220 may include a bending part 221, adapters 222, a coil spring 223, and an insertion tube 224. In this case, the bending part 221 may be disposed between the pair of adapters 222, one of the pair of adapters 222 may be coupled to the lighting/imaging part 210, and the other of the pair of adapters 222 may be coupled to the second coupling part 250.

The bending part 221 may be configured to be bent by the movement of the bending wire 230.

The bending part 221 includes first and second bending members 2211 and 2212 configured to rotate relative to one another. In this case, the bending part 221 may be configured such that the plurality of first bending members 2211 and the plurality of second bending members 2212 are disposed alternately. Further, an overall length of the bending part 221 may increase as the number of first bending members 2211 and the number of second bending members 2212 are increased. Therefore, an appropriate length of the insertion pipe 220 may be selected by adjusting the number of first bending members 2211 and the number of second bending members 2212 in accordance with a condition of use of the endoscope 1.

The first and second bending members 2211 and 2212 may be provided to rotate relative to each other.

The first bending member 2211 includes a first bending member body 2211a, concave portions 2211b, and wire through-holes 2211c.

The first bending member body 2211a may be formed in an annular shape as a whole. In this case, a thickness of the first bending member body 2211a in the longitudinal direction may repeatedly vary in a circumferential direction depending on an interval of a predetermined angle. For example, the first bending member body 2211a may be formed in a shape in which a thickness decreases as the distance from the concave portion 2211b increases in the circumferential direction.

Meanwhile, in the present embodiment, the first bending member body 2211a may be made of a resin material. For example, the first bending member body 2211a may be made of a plastic material. The above-mentioned material is advantageous in improving processability and reducing production costs.

In this case, the first bending member body 2211a may be formed with the concave portions 2211b so that the first bending member body 2211a may rotate relative to the second bending member 2212.

Four concave portions 2211b may be formed in the first bending member body 2211a. A pair of concave portions 2211b may be formed at one longitudinal end of the first bending member body 2211a, and a pair of concave portions 2211b may be formed at the other longitudinal end of the first bending member body 2211a. In this case, the pair of concave portions formed at one end of the first bending member body 2211a and the pair of concave portions formed at the other end of the first bending member body 2211a may be disposed alternately with a phase difference of 90 degrees in the circumferential direction. That is, the pair of concave portions 2211b formed at the ends based on the same direction may be disposed with a phase difference of 180 degrees in the circumferential direction, and the pair of concave portions 2211b formed at the ends based on different directions may be disposed with a phase difference of 90 degrees.

The concave portion 2211b may be recessed from a longitudinal end of the first bending member body 2211a. In this case, the concave portion 2211b may be recessed in an arcuate shape having a predetermined curvature from the longitudinal end of the first bending member body 2211a.

The concave portion 2211b may accommodate at least a part of a convex portion 2212b of the second bending member 2212 to be described below. In addition, the concave portion 2211b and the convex portion 2212b may rotate relative to each other. In this case, the concave portion 2211b and the convex portion 2212b may be in contact with each other.

Therefore, an interval between the first bending member body 2211a and a second bending member body 2212a may not be uniform in a state in which the first bending member body 2211a and the second bending member body 2212a are coupled. That is, when the first bending member 2211 and the second bending member 2212 are coupled, the concave portion 2211b and the convex portion 2212b may be in contact with each other, and an interval between the first bending member body 2211a and the second bending member body 2212a may increase as the distance from the concave portion 2211b and the convex portion 2212b increases in the circumferential direction.

With this interval, it is possible to provide a range in which the first bending member 2211 and the second bending member 2212 may rotate relative to each other in accordance with the movement of the bending wire 230.

Meanwhile, a radial thickness at a position on the first bending member body 2211a, at which the concave portion 2211b is formed, may increase. For example, the first bending member body 2211a may be formed to have a predetermined outer diameter on a concentric circle. However, an inner diameter at a position at which the concave portion 2211b is formed may decrease. That is, the first bending member body 2211a may be formed in a shape in which an inner peripheral surface at the position at which the concave portion 2211b is formed protrudes radially inward.

In addition, the wire through-hole 2211c may be formed in the longitudinal direction at the position at which the concave portion 2211b is formed. That is, the wire through-hole 2211c may be a circular hole formed at a position at which an inner peripheral surface of the first bending member body 2211a protrudes radially inward.

In this case, the bending wire 230 may pass through the wire through-hole 2211c.

The second bending member 2212 includes the second bending member body 2212a, the convex portions 2212b, and wire through-holes 2212c.

The second bending member body 2212a may be formed in an annular shape as a whole. In this case, a thickness of the second bending member body 2212a in the longitudinal direction may repeatedly vary in the circumferential direction depending on an interval of 180 degrees. For example, the thickness of the second bending member body 2212a in the longitudinal direction may decrease as the distance from a position, at which the convex portion 2212b is formed, increases in the circumferential direction.

Meanwhile, in the present embodiment, the second bending member body 2212a may be made of a resin material. For example, the second bending member body 2212a may be made of a plastic material. The above-mentioned material is advantageous in improving processability and reducing production costs.

In this case, the second bending member body 2212a may be formed with the convex portion 2212b so that the second bending member body 2212a may rotate relative to the first bending member 2211.

Four convex portions 2212b may be formed on the second bending member body 2212a. A pair of convex portions 2212b may be formed at one longitudinal end of the second bending member body 2212a, and a pair of convex portions 2212b may be formed at the other longitudinal end of the second bending member body 2212a. In this case, the pair of convex portions 2212b formed at one end of the second bending member body 2212a and the pair of convex portions 2212b formed at the other end of the second bending member body 2212a may be disposed alternately with a phase difference of 90 degrees in the circumferential direction. That is, the pair of convex portions 2212b formed at the ends based on the same direction may be disposed with a phase difference of 180 degrees in the circumferential direction, and the pair of convex portions 2212b formed at the ends based on different directions may be disposed with a phase difference of 90 degrees.

The convex portion 2212b may protrude from a longitudinal end of the second bending member body 2212a. In this case, the convex portion 2212b may be formed to correspond in shape to the concave portion 2211b. For example, the convex portion 2212b may protrude in an arcuate shape having a predetermined curvature from the longitudinal end of the second bending member body 2212a, and a curvature with which the convex portion 2212b protrudes and a curvature with which the concave portion 2211b is recessed may be equal to each other.

With this configuration, at least a part of the convex portion 2212b may be accommodated in the concave portion 2211b. In addition, the convex portion 2212b and the concave portion 2211b may rotate relative to each other while being in surface contact with each other.

Meanwhile, a radial thickness at a position on the second bending member body 2212a, at which the convex portion 2212b is formed, may increase. For example, the second bending member body 2212a may be formed to have a predetermined outer diameter on a concentric circle. However, an inner diameter at a position at which the convex portion 2212b is formed may decrease. That is, the second bending member body 2212a may be formed in a shape in which an inner peripheral surface at the position at which the convex portion 2212b is formed protrudes radially inward.

In addition, the wire through-hole 2212c may be formed in the longitudinal direction at the position at which the convex portion 2212b is formed. That is, the wire through-hole 2212c may be a circular hole at a position at which an inner peripheral surface of the second bending member body 2212a protrudes radially inward.

In this case, the bending wire 230 may pass through the wire through-hole 2212c.

Meanwhile, the inner peripheral surface of the convex portion 2212b may be formed to be equal in maximum inner diameter to the second bending member body 2212a without protruding radially inward. That is, the inner peripheral surface of the convex portion 2212b does not protrude radially inward from the position at which the wire through-hole 2212c is formed. Therefore, the entire inner peripheral surface of the second bending member 2212 has a uniform inner peripheral surface, and the inner peripheral surface at the position at which the wire through-hole 2212c is formed protrudes radially inward.

In addition, a wire guide groove 2212d may be formed in the inner peripheral surface of the convex portion 2212b. The wire guide groove 2212d may be recessed in the shape of a circular arc in the inner peripheral surface of the convex portion 2212b, and an origin of the arc may be connected to the wire through-hole 2212c.

With this shape, a movement range of a free end of the bending wire 230 may be restricted by the wire guide groove 2212d when the bending wire 230 moves in a state in which a position being in contact with the second bending member body 2212a is used as a fixed end. That is, a maximum angle at which each of the second bending members 2212 is bent may be a central angle of the arc of the wire guide groove 2212d.

The adapters 222 may be respectively coupled to two opposite longitudinal ends of the bending part 221. In this case, the adapter 222 disposed at one longitudinal side may be coupled to the lighting/imaging part 210, and the adapter 222 disposed at the other longitudinal side may be coupled to the second coupling part 250.

The adapter 222 includes an adapter body 2221, a spring coupling portion 2222, a wire accommodation groove 2223, a wire through-hole, and a bending member connection portion 2225.

The adapter body 2221 may be formed in a cylindrical shape as a whole, and the spring coupling portion 2222 may be formed on an outer peripheral surface of the adapter body 2221. In addition, the wire accommodation groove 2223 may be formed in the longitudinal direction in an inner or outer peripheral surface of the adapter body 2221. In addition, the wire through-hole may be formed in the longitudinal direction in the adapter body 2221. In addition, the bending part connection portion 2225 may be formed at a longitudinal end of the adapter body 2221.

A longitudinal end of the coil spring 2223 may be coupled to the spring coupling portion 2222. For example, the spring coupling portion 2222 may have a rib shape protruding in the circumferential direction from the outer peripheral surface of the adapter body 2221. Therefore, the end of the coil spring 2223 may be caught and supported by the spring coupling portion 2222.

Therefore, the coil spring 223 may be disposed between the spring coupling portions 2222 formed on the pair of adapters 222, and a restoring force of the coil spring 223 may be applied to the pair of adapters 222.

The wire accommodation groove 2223 may be formed in the longitudinal direction in the inner or outer peripheral surface of the adapter body 2221. For example, four wire accommodation grooves 2223 may be formed in the longitudinal direction in the inner peripheral surface of the adapter body 2221.

At least a part of the bending wire 230 may be accommodated in the wire accommodation groove 2223. The bending wire 230 may reciprocate in the longitudinal direction along the wire accommodation groove 2223 in accordance with the operation of the manipulation knob 120.

Although not illustrated, the wire through-hole may be formed in the longitudinal direction of the adapter body 2221. In this case, at least a part of the inner peripheral surface of the adapter body 2221 may protrude from the position at which the wire accommodation groove 2223 is formed, and the wire through-hole may be formed in the protruding portion. That is, the wire through-hole may be disposed at the position connected to the wire accommodation groove 2223.

In this case, the bending wire 230 may pass through the wire through-hole.

Meanwhile, the bending member connection portion 2225 may be formed to be coupled to be rotatable relative to the bending part 221. That is, the bending member connection portion 2225 may be formed with a convex or concave structure corresponding to the concave portion 2211b of the first bending member 2211 or the convex portion 2212b of the second bending member 2212 so that the bending member connection portion 2225 may be coupled to the concave portion 2211b of the first bending member 2211 or the convex portion 2212b of the second bending member 2212.

Meanwhile, the coil spring 223 may be disposed to surround an outer peripheral surface of the bending part 221. That is, the coil spring 223 may be disposed in a shape that surrounds outer peripheral surfaces of the first and second bending members 2211 and 2212 in the state in which the first bending member 2211 and the second bending member 2212 are coupled.

In contrast, two opposite longitudinal ends of the coil spring 223 may be coupled to the adapters 222. The coil spring 223 may elastically support the spring coupling portions 2222 of the pair of adapters 222. Therefore, in case that the bending part 221 is bent by the manipulation of the manipulation knob 120, the coil spring 223 may apply an elastic force to restore the bending part 221 to an original position.

Meanwhile, the insertion tube 224 may be disposed at the other longitudinal side of the insertion pipe 220. The insertion tube 224 may be formed in the shape of a bendable tube, and the bending wire 230, the channel 240, and the terminal may pass through the inside of the insertion tube 224. Although not illustrated, a coil pipe may be provided in the insertion tube 224 and provide an elastic force to the insertion tube 224.

Meanwhile, the bending wire 230 may receive a manipulation force of the manipulation knob 120 through the second coupling part 250 and bend the insertion pipe 220 by means of a rectilinear reciprocating motion.

One longitudinal end of the bending wire 230 may be coupled to the adapter 222 or the lighting/imaging part 210, and the other longitudinal end of the bending wire 230 may be coupled to the second wire frame 256. In this case, the bending wire 230 may pass through the wire through-hole of the adapter 222, the wire through-hole 2211c of the first bending member 2211, and the wire through-hole 2212c of the second bending member 2212.

Therefore, at a side at which the bending wire 230 is pulled, the first bending member 2211 and the second bending member 2212 may rotate relative to each other so that an interval between the first bending member 2211 and the second bending member 2212 decreases. In contrast, at a side at which the bending wire 230 is unwound (extended), the first bending member 2211 and the second bending member 2212 may rotate relative to each other so that the interval between the first bending member 2211 and the second bending member 2212 increases. Therefore, the first bending member 2211 and the second bending member 2212 may define a bent shape. In this case, a bending angle between the first bending member 2211 and the second bending member 2212 may be restricted in accordance with an angle of the wire guide groove 2212d.

Meanwhile, with reference to FIGS. 4 to 13, the second coupling part 250 is detachably coupled to the first coupling part 150 of the manipulation part 100. The second coupling part 250 includes the second coupling part bodies 251, the second coupling plates 255, and the second wire frames 256.

One longitudinal side of the second coupling part body 251 may be coupled to the insertion pipe 220, and the other longitudinal side of the second coupling part body 251 may be coupled to the first coupling part 150. In this case, one longitudinal side of the second coupling part body 251 may be coupled to the other longitudinal side of the insertion pipe 220, and the other longitudinal side of the second coupling part body 251 may be detachably coupled to the first coupling part body 151.

The second coupling part body 251 may be formed in a shape having a diameter that decreases toward one longitudinal end (leading end). As a result, one longitudinal end of the second coupling part body 251 may be formed in a shape capable of being coupled to the insertion tube 224 of the insertion pipe 220 and connected to the insertion tube 224 of the insertion pipe 220.

The other longitudinal side of the second coupling part body 251 may be provided to engage with and be coupled to the first coupling part 150. In one example, the other longitudinal side of the second coupling part body 251 may be formed in the form of a plate having three sides extending toward the manipulation part 100. In another example, the other longitudinal side of the second coupling part body 251 may be provided in the form of a pair of plates extending toward the manipulation part 100. Therefore, the second coupling part bodies 251 may be coupled to the first coupling part bodies 151 of the first coupling part 150.

As a result, the second coupling part bodies 251 may be fitted with the first coupling part bodies 151. That is, the second coupling part body 251 may be coupled to the first coupling part body 151 and accommodate therein the first coupling plate 155, the first wire frame 156, the second coupling plate 255, and the second wire frame 256.

The second coupling plates 255 may be disposed between a pair of surfaces of the second coupling part bodies 251 that face each other. The second coupling plates 255 may be disposed inside the second coupling part bodies 251 and disposed at positions that face the second coupling part bodies 251.

In addition, the second coupling plate 255 may be disposed at positions that face the first coupling plates 155. In this case, when the first coupling plate 155 moves in accordance with the movement of the rack gear 154, the first coupling plate 155 may be coupled to the second coupling plate 255.

For example, the second coupling plate 255 may be formed in the shape of a quadrangular plate having a predetermined thickness. In this case, fixing portions 255a may be formed on the second coupling plates 255 to fix the state in which the first coupling plates 155 are coupled to the second coupling plates 255. In one example, the fixing portion 255a may be formed in the shape of a protrusion and coupled to the fixing portion 155a formed in the shape of a hole formed in the first coupling plate 155.

Meanwhile, the second wire frames 256 may be disposed in the second coupling plates 255. The second wire frame 256 may be coupled to the second coupling plate 255 and configured to rectilinearly reciprocate. For example, the second coupling plate 255 may be formed with a guide groove that guides the rectilinear movement of the second wire frame 256, and the second wire frame 256 may be disposed in the guide groove.

Meanwhile, the bending wire 230 may be coupled to the second wire frame 256. For example, the bending wire 230 may be coupled to one longitudinal side of the second wire frame 256, and the second wire frame 256 may be configured to rectilinearly reciprocate in the longitudinal direction.

Meanwhile, the first wire frame 156 may be coupled to the second wire frame 256. For example, the frame coupling part 256b may be formed at the other longitudinal side of the second wire frame 256. The frame coupling part 256b may have a shape in which a plurality of ribs and a plurality of grooves are alternately formed. The frame coupling part 256b may be shaped to correspond to the frame coupling part 156b of the first wire frame 156. That is, the first wire frame 156 and the second wire frame 256 may be coupled in a state in which the first wire frame 156 and the second wire frame 256 engage with each other.

An assembly obtained by coupling the first wire frame 156 and the second wire frame 256 may be disposed in the configuration in which the first coupling plate 155 and the second coupling plate 255 are coupled. In this case, the first wire frame 156 may be accommodated in the guide groove formed in the first coupling plate 155, and the second wire frame 256 may be accommodated in the guide groove formed in the second coupling plate 255. Therefore, when the first coupling plate 155 and the second coupling plate 255 are coupled, the first coupling plate 155 and the second coupling plate 255 may surround the assembly obtained by coupling the first wire frame 156 and the second wire frame 256. That is, the assembly obtained by coupling the first wire frame 156 and the second wire frame 256 may reciprocate in the longitudinal direction (forward/rearward direction), but four sides, i.e., upper, lower, left, and right sides of the assembly may be restricted by the first coupling plate 155 and the second coupling plate 255.

Therefore, the manipulation wire 130 coupled to the first wire frame 156 and the bending wire 230 coupled to the second wire frame 256 may move in conjunction with each other, and the insertion pipe 220 may be bent by the manipulation of the manipulation knob 120.

While the present invention has been described with reference to the specific embodiments, the specific embodiments are only for specifically explaining the present invention, and the present disclosure is not limited to the specific embodiments. It is apparent that the present invention may be modified or altered by those skilled in the art without departing from the technical spirit of the present invention.

All the simple modifications or alterations to the present invention fall within the scope of the present invention, and the specific protection scope of the present invention will be defined by the appended claims.

## Claims

1. An endoscope comprising:
an insertion part provided with a lighting/imaging part and an insertion pipe; and
a manipulation part provided with a manipulation knob and configured to bend the insertion pipe in accordance with a manipulation of the manipulation knob,
wherein the insertion pipe comprises a bending part configured to be bent in accordance with a manipulation of the manipulation part, and
**characterized in that** the bending part comprises:
a first bending member formed with a concave portion; and
a second bending member formed with a convex portion and fastened to the first bending member.

2. The endoscope of claim 1, wherein the first bending member comprises an annular first bending member body formed with the concave portion, and
wherein the concave portion is provided as a pair of concave portions respectively formed at two opposite longitudinal ends of the first bending member body.

3. The endoscope of claim 2, wherein the pair of concave portions are formed at positions that face each other in a circumferential direction at the two opposite longitudinal ends of the first bending member body.

4. The endoscope of claim 2, wherein the concave portions are disposed with a predetermined phase difference in a circumferential direction of the first bending member body.

5. The endoscope of claim 1, wherein the second bending member comprises a second bending member body having a longitudinal end from which the convex portion protrudes, and
wherein the convex portion is relatively rotatably accommodated in the concave portion.

6. The endoscope of claim 1, wherein the insertion part further comprises a bending wire configured to bend the insertion pipe while rectilinearly reciprocating, and
wherein the second bending member further comprises a wire guide groove formed in an inner surface of the convex portion and configured to restrict a bending angle of the bending wire.

7. The endoscope of claim 5, wherein the insertion part further comprises a bending wire configured to bend the insertion pipe while rectilinearly reciprocating, and
wherein a wire through-hole through which the bending wire passes is disposed radially inside the concave portion or the convex portion.

8. The endoscope of claim 1, wherein the insertion pipe further comprises adapters coupled to the first bending member or the second bending member and disposed at a longitudinal end of the bending part.

9. The endoscope of claim 8, wherein the insertion part further comprises a bending wire configured to bend the insertion pipe while rectilinearly reciprocating, and
wherein the adapter comprises:
an adapter body formed in a cylindrical shape; and
a wire accommodation groove formed in a longitudinal direction of the adapter body and configured to accommodate at least a part of the bending wire.

10. The endoscope of claim 1, wherein the insertion pipe further comprises a coil spring configured to accommodate therein the bending part.

11. The endoscope of claim 10, wherein two opposite longitudinal ends of the coil spring are coupled to adapters coupled to two opposite longitudinal ends of the bending part.

12. An endoscope comprising:
an insertion part provided with a lighting/imaging part and an insertion pipe; and
a manipulation part provided with a manipulation knob and configured to bend the insertion pipe in accordance with a manipulation of the manipulation knob,
**characterized in that** the insertion pipe comprises a bending part configured to be bent in accordance with a manipulation of the manipulation part, and
wherein the bending part comprises a first bending member and a second bending member that are alternately disposed in a coil spring and configured to rotate relative to each other.
